# EUROPEAN PATENT SPECIFICATION

(11) **EP 1 309 330 B1**
(45) Date of publication and mention of the grant of the patent: **26.03.2008**
(21) Application number: 01952201.0
(22) Date of filing: 22.06.2001
(51) Int. Cl.: A61K 31/5415, A61K 41/00, A61K 47/48, A61P 35/00

(54) **PHOTOSENSITIZERS WITH LIGAND TARGETING PROPERTIES FOR TUMOR THERAPY**
PHOTOSNESIBILISIERENDESUBSTANZENMIT LIGAND-TARGETING EIGENSCHAFTEN ZUR TUMORTHERAPIE
PHOTOSENSIBILISANTS POUR TH RAPIE ANTITUMORALE CAPABLES DE CIBLAGE LIGANTAIRE

(30) Priority: 22.06.2000 US 599660; 21.12.2000 US 745458
(43) Date of publication of application: 14.05.2003
(73) Proprietor: CeramOptec GmbH, 53121 Bonn (DE)
(72) Inventor: MOSER, Joerg, G., 40591 Dusseldorf (DE)
(86) International application number: PCT/US2001/020086
(87) International publication number: WO 2001/097814

(56) References cited:
- WO-A-01/51524
- US-A- 5 070 081
- MOSER J G ET AL: "Cyclodextrin Dimers Used to Prevent Side Effects of Photochemotherapy and General Tumor Therapy" JOURNAL OF INCLUSION PHENOMENA AND MOLECULAR RECOGNITION IN CHEMISTRY, KLUWER, DORDRECHT, NL, vol. 25, 1996, pages 29-34, XP002097236 ISSN: 0923-0750
- RUEBNER, A. ET AL: "Carrier systems in PDT II: Accumulation strategies of biotin-avidin coupled photosensitizers developed on cultured tumor cells" PROCEEDINGS OF SPIE-THE INTERNATIONAL SOCIETY FOR OPTICAL ENGINEERING , 2625(PHOTOCHEMISTRY: PHOTODYNAMIC THERAPY AND OTHER MODALITIES), 328-332 CODEN: PSISDG; ISSN: 0277-786X, 1996, XP008037924
- HIRTH, ANDREAS ET AL: "New biotinylated phthalocyanines for the photodynamic therapy of cancer" PROCEEDINGS OF SPIE-THE INTERNATIONAL SOCIETY FOR OPTICAL ENGINEERING , 3191(PHOTOCHEMOTHERAPY: PHOTODYNAMIC THERAPY AND OTHER MODALITIES III), 309-314 CODEN: PSISDG; ISSN: 0277-786X, 1997, XP008037922
- CLINE ET AL.: 'beta-Cyclodextrin inclusion complexes with alpha-dlimineruthenium (II) photosensitizers' J. PHYS. CHEM. vol. 89, no. 1, 1985, pages 94 - 97, XP002947613
- LOUKAS ET AL.: 'Characterization and photoprotection studies of a model gamma-cyclodextrin-included photolabile drug entrapped in liposomes incorporating light absorbers' J. PHYS. CHEM. vol. 99, no. 27, 1995, pages 11035 - 11040, XP002947614
- RUEBNER ET AL.: 'A cyclodextrin dimer with a photocleavable linker as a possible carrier for the photosensitizer in photodynamic tumor therapy' PROC. NATL. ACAD. SCI. USA vol. 96, no. 26, 21 December 1999, pages 14692 - 14693, XP002947615

## Description

The present invention relates to photodynamic therapy and chemotherapy, wherein a "parachute" structure is used to deliver a therapeutic agent to a defined subcellular location, and therefore the action of the therapeutic agent, such as destroying tumor cells, is achieved more effectively.

During the last two decades, there has been an increasing interest in utilizing photosensitizers for cancer therapy, where the technique is known as Photodynamic Therapy (PDT). Dougherty TJ, Photosensitizers: Therapy and detection of malignant tumors, Photochem. Photobiol, 45(6): 879-889, (1987) and Dougherty TJ, Photodynamic therapy, Photochem. Photobiol, 58(6): 895-900, (1993) describe a technique where singlet oxygen, oxygen radicals, and superoxides/peroxides are produced by *in situ* photosensitization of previously applied chromophores, and thus destroy the malignant cells. The technique utilizes non-toxic, photosensitizing drugs in combination with non-hazardous irradiation, and has the potential of being more selective yet no less effective when compared with the commonly used chemotherapy or radiotherapy. It is therefore expected to increase the quality of life for treated patients. Moreover, Sokolov V. et al., Multicourse PDT of malignant tumors: the influence of the primary tumor, metastatic spreading and homeostasis in cancer patients, SPIE Biomedical Optics 3191, 322-329 (1996) shows that no scars are observed after removal of the tumors using this technique and the surrounding muscular structures are fully functional.

The photosensitizers used in PDT need to have a high quantum yield for singlet oxygen production, as well as characteristics of high affinity and selectivity for the malignant tissue. Porphyrins have a high quantum yield for the formation of an excited triplet state. The difference between the energies of triplet state and ground-state oxygen makes them good energy donors to transfer its energy to the ground state to form singlet oxygen. One experimental drug known as Photofrin II (a purified version of hematoporphyrin derivative) is currently involved in randomized clinical trials. Other photosensitizing drugs used in photodynamic therapy procedures include phthalocyanines, merocyanine 540, substituted purines, xanthenes (Rhodamine 123), cationic cyanine dyes, chlorines, chalcogenapyrylium dyes containing selenium or tellurium atoms in the chromophore, phenothiazinium derivatives, benzophenoxoniums (Nile Blue A) and triarylmethanes (Methylene Blue, Victoria Blue BO [VB-BO]).

Illumination of the target site by an appropriate light source, such as a sunlamp, an argon-pumped dye laser, or more recently, diode lasers, induces the cytotoxic effect on the cells of the target site by one of two proposed mechanisms. In Type I photosensitization, the electronically excited drug reacts directly with a biological substrate, forming radicals which can initiate subsequent radical reactions that induce cytotoxic damage. Type II photosensitization involves energy transfer from the electronically excited drugs to ground state oxygen, producing singlet oxygen which subsequently produces cytotoxic oxygenated products destroying the cell membrane of tumor (and vascular endothelial) cells directly.

Although remarkable results have been obtained in some PDT trials, several problems remain. The low solubility of some of the sensitizers reduces their usefulness for intravascular administration because it can provoke thromboembolic events. The use of liposomes as transport vehicles can overcome the problem of the solubility, but there still remains the difficulty of directing the sensitizers to specific target sites. Moreover, liposomes administered intravenously to subjects are rapidly accumulated in the reticuloendothelial system often inducing several severe allergic reactions. High liposome concentration is thereby rapidly achieved in organs with fenestrated capillaries, such as the liver, spleen, and bone marrow. Liposomal systems can be effective in treating tumors that infiltrate these organs (such as hematological malignancies), but have been less useful in treating targeted tumors in other anatomical locations.

Very high systemic doses of the sensitizer must often be given to achieve therapeutic levels at irradiated tumor sites, hence many sites in the body arc nonselectively infiltrated by the sensitizer. As the amount of the applied photosensitizer increases, the chances of its accumulation in normal tissues and the accompanying risk of damaging non-malignant sites profoundly increases.

There have been many attempts to increase the specificity of the photosensitizers for the tumor and thereby concentrating a sufficient amount of molecules in the tumor cells. Moser JG "Definitions and General Properties of 2nd and 3rd Generation Photosensitizers and "Carrier and Delivery Systems" in Photodynamic Tumor Therapy - 2nd and 3rd Generation Photosensitizers, pp. 3-8 and 127-136 respectively, [Moser, JG ed, Harwood Academic Publishers, London, (1998)] describe an approach of the coupling of several molecules of the dye with tumor specific antibodies which resulted in excellent photo toxicity *in vitro.* However, *in vivo* this method failed due to the recognition of the vast complex by the reticuloendothelial system, subsequent accumulation in the liver, and digestion of the compound before it reached the target site. Hirth A et al., New biotinylated phthalocyanines for the photodynamic therapy of cancer, SPIE Biomed. Optics 3191, 309-314, (1997) disclosed a more promising approach using polyphasic tumor targeting. Here, the antibodies are coupled with only low molecular weight substituents like biotin, so that they are not recognized by the reticuloendothelial system. After clearing the excessive biotinylated antibodies in the system, the photosensitizer coupled to avidin can be administered and accumulate at the cells labeled by the antibody. This method still has to be developed further for *in vivo* use in tumor therapy. But even if the targeting of the dye to the tumor would be successful, still an amount of at least 10⁷ photosensitizer molecules per cell has to be reached for the effective destruction of the tumor, which none of the described techniques have successfully achieved.

The reduction of the amount of photosensitizer molecules necessary for successful therapy could be achieved by more selective targeting of the dyes to the sites in the cell, where the compounds can develop the most effective action for the destruction of the malignant cells. Specific subcellular targeting of photosensitizers was only rarely performed and observed. One example is Rhodamine 123 targeting mitochondrial membranes. Matz et al, Fluorescent proteins from nonbioluminescent Anthozoa species, Nature Biotechnol, 17(10): 969-73, (1999) describes red-fluorescent targeting proteins for achieving the same effect. Kessel D, Use of fluorescent probes for characterizing sites of photodamage, The Spectrum 6(2), 1-6, (1993) describes other examples and techniques of detection. The frequent localization of bacteriochlorophyll derivatives is at the Golgi apparatus. However, both localizations are not very deleterious for cancer cells since these cells obtain their energy from cytoplasmic basal metabolism.

In summary, there are various attempts to increase the specificity of photo therapeutic drugs and thereby to reduce the doses necessary for the successful photodynamic therapy of tumors. However, no satisfying solution to this problem has been presented so far. The present invention provides a novel method to enhance the effect of photosensitizers or other therapeutic compounds on cells to achieve successful therapy with reduced doses of the drugs.

It is an object of the present invention to provide a system for and method of localizing therapeutic compounds at the most sensitive sites in a cell, thereby reducing therapeutic doses of the drug and in turn reducing the side effects of the therapy.

It is another object of the present invention to localize therapeutic compounds at cell membranes which results in a reduction of the dosage needed by a factor of 10-100 or more while achieving the same therapeutic effect.

It is a further object of the present invention to localize therapeutic compounds not only at a cell membrane, but also at defined distances to the membrane within the cells to optimize the destroying properties of the respective drug.

Still another object of the present invention is to modify the hydrophilic residues of the delivery structure so that it can also carry signals for targeting of the therapeutic complex to a specific tissue type.

Briefly stated, the present invention provides a drug delivery system wherein a "parachute" structure is coupled to a therapeutic compound. The "parachute" structure comprises hydrophilic branched molecules with a defined action diameter, where action diameter is the distance between the hydrophilic moieties that is necessary to localize the complex in the cell membrane. The complex (a parachute structure coupled with a therapeutic compound) is either fixed at a cell membrane or delivered to a defined distance from the membrane within the cell. The membrane-anchoring/localizing effect of the parachute is achieved by hydrophilic structures linked with a branching unit of desired therapeutic compounds (such as photosensitizers and chemotherapeutics). For example, di-glucosamine, a hydrophilic structure of a defined action diameter, functions to retard the molecules at the cell membrane, and thus avoids or slows deeper penetration of the therapeutic compounds into the cell during the short time span before irradiation. The defined action diameter can be achieved by using a branching unit, which can be triazine trichloride or trimesinic acid trichloride, to connect the therapeutic compound such as porphyrin over a diamide spacer bridge with two glucosamine residues. The hydrophilic moiety can also consist of a cyclodextrin attached to the drug. The attached cyclodextrin renders the complex water soluble and is bulky and hydrophilic enough to retain the complex in the membrane. Furthermore, the parachute structures can be connected by a spacer (e.g. β-amino acids, γ-amino butyric acid, poly-amino acids, aliphatic, aromatic or heterocyclic molecules) instead of directly binding to the therapeutic compound, so that the therapeutic compounds can be localized within the cells at a defined distance from the cell membrane. A spacer containing a breaking point (for example, it can be cleaved by the action of cellular enzymes) can determine the time span, during which the drug exhibits its therapeutic activity. The hydrophilic residues can also carry signals for targeting the parachute-therapeutic complex to a defined tissue type. This can be mediated by an antibody which is specific for a tumor marker. Alternatively, a biotin can be attached at C6 position of the sugar and then react with an avidin-labeled tumor-specific antibody. The biotin-avidin system has the advantage of minimizing the size of the delivery structure so that it will not be recognized by the reticuloendothelial system and get destroyed. The parachute function may also be achieved by other, more bulky hydrophilic structures such as oligosaccharides connected to the branching unit. Such sugar oligomers have specific attachment points to cell selectins, and therefore do not need additional molecular structures to target a specific tumor tissue. The use of the parachute structure gives the advantage of being able to localize a photosensitizer or chemotherapeutic drug at the site within a cell where it can destroy the tumor cell most effectively. This reduces the level of necessary systemic doses of the drugs, promotes drug excretion, and therefore considerably reduces side effects of the therapy.

The above, and other objects, features and advantages of the present invention will become apparent from the following description read in conjunction with the accompanying drawings.

Fig. 1 shows a general embodiment of a drug delivery system.

Fig. 2 illustrates an example of a photosensitizer coupled by a spacer to a parachute structure consisting of a branching unit carrying two glucosamine residues.

Fig. 3 demonstrates exclusive location of a modified photosensitizer at the cell membrane (fluorescence).

The plasma membrane of the cell is a selective barrier for the influx of external substances in the cell. Moreover, the cell membranes have a very high condensor potential, expressed as a field strength = 105 V/cm. Consequently, disturbances at the cell membrane are highly efficient for the destruction of the whole cell. The present invention achieves exclusive localization of a therapeutic compound at a cell membrane or at a defined distance from the membrane within the cell by linking the drug to a parachute structure. Hydrophilic branched molecules such as di-glucosamine function as a parachute because of their hydrophilic nature and the distance between the molecules (action diameter). Such characteristics of the hydrophilic branched molecules will keep them from entering a cell while a drug attached to them penetrates the cell. Since the parachute is built up in a way that the distance between the hydrophilic branched molecules and the drug can vary as desired by adding different spacer molecules, the drug is delivered to a cell either very near to the cell membrane (when there is no spacer molecules attached) or at a defined distance from the cell membrane within the cell depending on the type and number of spacer molecules used. Spacer molecules can be β-aminoacids, γ-amino butyric acid, poly-amino acids such as poly-lysine, poly-serine, poly-threonine, or aliphatic, aromatic or heterocyclic molecules. Most preferred are molecules containing a cyclodextrin as a parachute structure. Cyclodextrins display the necessary characteristics to render the attached drug water soluble and localizing it at the cell membrane, since cyclodextrins are bulky sugar structures which do not need a branching unit to achieve the necessary action diameter. The functional groups of cyclodextrins easily allow attachment of the drug with or without spacer structures in between.

Different targeting signals (such as antibodies or biotin) can be attached to the hydrophilic branched molecules thus directing the entire complex to a specific tissue type such as a tumor or cancer. The hydrophilic branched molecules can also be sugar oligomers with specific attachment points at cell selectins for targeting purposes.

The parachute structure is particularly useful when the therapeutic compound is a photosensitizer, such as a porphyrin, which is commonly used in photodynamic therapy. The parachute structure will hold photosensitizers near the cell membrane during radiation activation so that the drug is much more effective. The dosage of photosensitizer needed when using the parachute structure is reduced by a factor of about 100 or more to achieve the same effect as when the drug is used in the conventional way. Porphyrin molecules containing only one propionic acid residue can be attached to the hydrophilic branched molecules through a branching unit, such as triazine trichloride or trimesinic acid trichloride. Moreover, the present invention has advantages when the therapeutic compound attached to the parachute is a chemotherapeutic drug like Merphalane, which needs membrane contact for specific functioning and has serious side effects on the liver function.

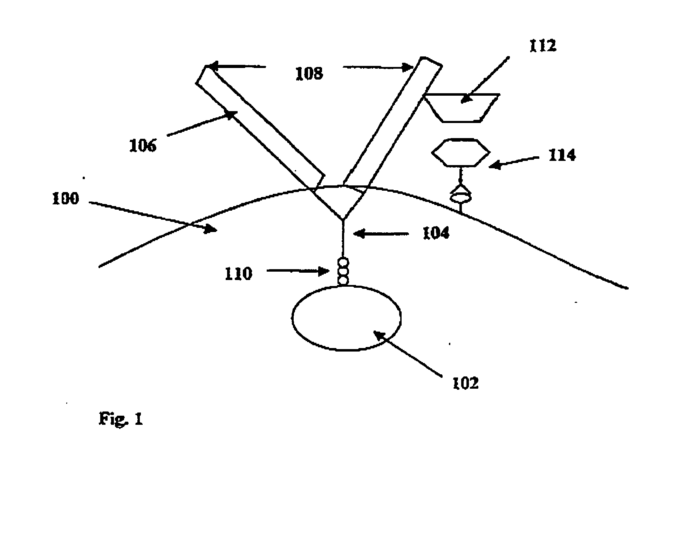

Fig. 1 demonstrates a general scheme of a drug delivery system. According to the present invention, the system is constructed by attaching therapeutic compound **102** to a parachute structure. The parachute structure comprises hydrophilic branched molecules **106** with defined action diameter **108.** The parachute structure can be directly linked to therapeutic compound **102,** or it can be linked by branching unit **104,** or branching unit **104** plus spacer **110** to therapeutic compound **102.** The parachute structure can also be modified to target specific cell type such as cancer cell **100.** For example, biotin **112** can be attached to the parachute structure and reacts to avidin-labeled tumor-specific antibody 114.

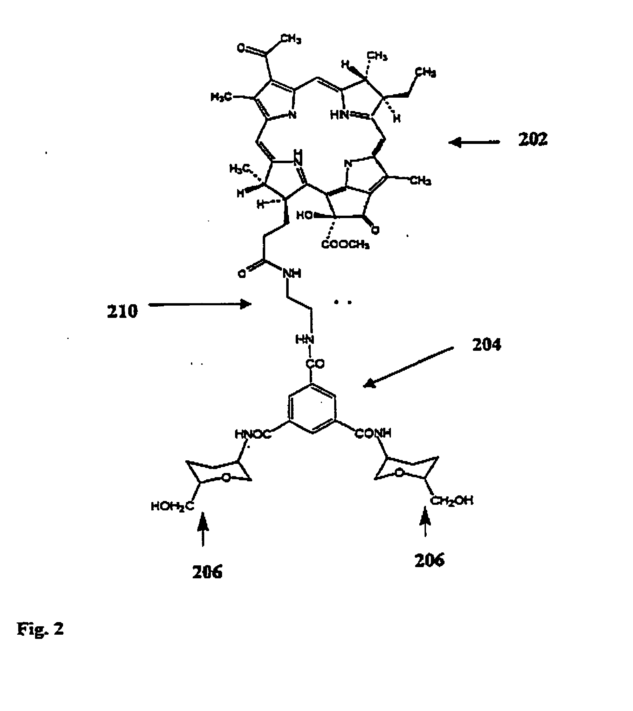

Fig. 2 shows the hydrophilic moiety of parachute structure consists of two glucosamine residues 206. The parachute effect is achieved by the linkage of aminosugars to branching unit 204, triazine trichloride or trimesinic acid trichloride. Spacers 210 can be linked to branching unit 204. Spacer 210 can be β-aminoacids, γ-amino butyric acid, or poly-amino acids. Also spacers with preformed breaking points, e.g. amino acid sequences recognized by cellular protcases, could be used. As an example of therapeutic agents, OH-bacteriopheophorbide 202 is used, which is connected at the propionic acid side chain at C17 with an ethyl diamine group and further with the parachute structure. The free amino group of the resulting amide reacts readily with branching unit 204. The glucosamine residues can be modified to mediate the tumor specific targeting of the delivery system.

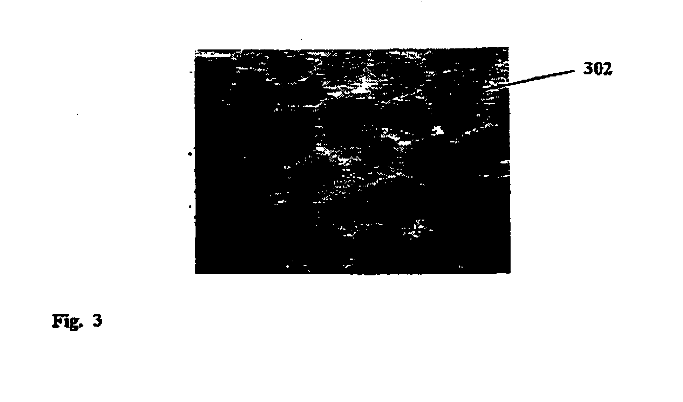

Fig. 3 illustrates the detection of the complex at the cell surface by fluorescence microscopy. Fluorescence is induced on the cells with green light (546nm), and the localization of complex **302** is observed at the cell membrane.

The present invention is further illustrated by the following examples, but is not limited thereby.

### Example 1: Construction of a branched sugar moiety parachute with therapeutic compound.

a. Bacteriopheophorbide (therapeutic compound) is activated in DMF by incubation with 1.1 Mol. Eq. di-isopropyl carbodi-imide at 0°C for 30 -60 min. This mix is added dropwise to a solution of 5 Mol. Eq. diaminoethane in DMF, dissolved under addition of 1.1 Mol. Eq. dimethyl amino pyridine. The product is isolated by ion exchange chromatography on CM-Cellulose or SP-Sepharose in DMF using a formate gradient.
b. Addition of the spacer. A solution of triazine trichloride in toluene is centrifuged to remove insoluble matter and titrated at room temperature with 2 Mol. Eq. of β-alanine or γ-amino butyric acid in the presence of K₂CO₃. The reaction proceeds overnight. The white precipitate is dissolved in methanol and reacted with the product of (1) at 85°C.
c. Synthesis of the branched glucosamine moiety. The product (2) is purified by ion exchange chromatography on DEAE-Sephadex or QAC-Sepharose in methanol-formiate gradients and freeze-dried. After activation by di-isopropyl carbodi-imide in DMF, a watery solution of glucosamine is added. Alternatively, a solution of methyl-glucosamine in 2-methoxy ethanol is added without activation.

### Example 2: Construction of a cyclodextrin parachute with therapeutic compound

a. OH-Bacteriopheophorbide is prepared in the usual manner, slightly acidified, and reacted first with an equimolar amount of di-isopropyl carbodi-imide. After half an hour N-hydroxy succinimide is added in equimolar amount and reacted over night.
b. Hydroxypropyl-γ-cyclodextrin (Molecusol^{™}) is dissolved in pyridine and reacted with toluene sulfonyl chloride in triple-molar amount. The mixture reacts for 3 hours at room temperature and is freeze-dried. The product is dissolved in water containing 10fold excess of diamino propane. After incubation overnight the product is freeze-dried again.
c. Products from, A and # B are mixed in equimolar amounts. Finally, biotin-N-hydroxy succinimide is added in sufficient excess (more than 2 mole equivalents) to obtain the desired product: di-biotinyl hydroxy bacteriopheophorbide amidopropylamide gamma-cyclodextrin.

### Example 3: Treatment of patients with the substance and PDT

For a patient, a dose of 0.8 - 1.0 mg/kg of the product should be sufficient for intravenous injection. Irradiation should follow at 760 nm and after only 15 min. The light power should be reduced to 200 mW , and no more than 20 J/cm² should be applied. The persistence of the dye in blood can be followed by fluorescence spectrometry and a reduction by a factor of 10 should be observed after 1 week.

## Claims

1. A complex for delivery and application of drugs to cell membranes or a defined distance from the membrane within cells comprising:
a parachute structure **(106),** having a defined action diameter **(108)** which hinders said structure from penetrating through a cell **(100)** membrane;
wherein said parachute structure comprises hydrophilic moieties which are sugar residues having said action diameter **(108);** and
a therapeutic compound **(102),** which can penetrate said cell **(100)** membrane while remaining a part of said complex.

2. A complex according to claim 1, wherein said action diameter can be achieved by
a branching unit to which said hydrophilic moieties are bound, or
glucosamine molecules attaching to said branching unit, or
monomer / oligomer sugar residues with specific attachment points to selectins on specific cells so that the complex is targeted to said specific cells, or
a cyclodextrin.

3. A complex according to claims 1 or 2, wherein said therapeutic compound is a photosensitizer or a chemotherapeutic drug, wherein said photosensitizer is preferably close to said membrane during time of activation to render said photosensitizer more effective compared to a similar photosensitizer without said parachute structure.

4. A complex according to any one of claims 1-3, wherein said parachute structure is directly bound to said therapeutic compound or connected with said therapeutic compound by a spacer, wherein said spacer is preferably β-aminoacids, γ-amino butyric acid, or poly-aminoacids, and wherein type and number of said spacer used define the distance of said therapeutic agent to cell membranes or its localization within the cell.

5. A complex according to claim 4, wherein said spacer is preferably an aliphatic, aromatic or heterocyclic molecule, or an amino acid sequence.

6. A complex according to claim 5, wherein said amino sequence has an enzyme cleavable breaking point.

7. A complex according to any one of claims 4 - 6, wherein using different number or type of said spacers to connect said therapeutic compound and said parachute structure delivers said complex into subcellular compartments at a defined distance from surface of said compartments.

8. A complex according to any one of claims 1 - 7, wherein said parachute structures are modified with signals for targeting said complex to a defined tissue or cell type in an organism.

9. A complex according to claim 8, wherein said modified signals contain bridging structures like a biotin-avidin system.

10. A complex according to any one of claims 1-9, wherein said complex can be used for destruction of cells, and wherein said cells are prokaryotic, preferably bacteria, or wherein said cells are eukaryotic, preferably human and animal cells.

11. A use of a complex having at least one parachute structure and at least one photosensitizer in the manufacture of a medicament for the treatment and selective destruction of eukaryotic or prokaryotic cells comprising the steps of:
a. administering said complex, wherein said complex contains a parachute structure and a photosensitizer; and
b. waiting for a interval to allow said complex to selectively localize at cell membranes or at a defined position within a cell; and
c. irradiating a region where said complex was administered for a defined time interval and intensity to activate said photosensitizer, wherein said time interval and intensity are sufficient to achieve selective destruction of desired cells.

## Patentansprüche

1. Ein Komplex zum Transport und zur Applikation von Wirkstoffen an Zellmembranen oder in einer bestimmten Distanz von Membranen im Zellinnern, bestehend aus:
Einer Fallschirmstruktur **(106)** mit einem bestimmten Aktionsdurchmesser **(108),** der die besagte Struktur daran hindert, die Membran einer Zelle **(100)** zu durchdringen;
Wobei die besagte Fallschirmstruktur hydrophile Reste einschließt, bei denen es sich um Zuckerreste mit besagtem Aktionsdurchmesser **(108)** handelt; und
einer therapeutischen Verbindung **(102),** welche die Membran der besagten Zelle **(100)** durchdringen kann und gleichzeitig Teil des besagten Komplexes bleibt.

2. Ein Komplex nach Anspruch 1, wobei der besagte Aktionsdurchmesser erreicht werden kann durch
Eine Verzweigungseinheit, an die besagte hydrophile Reste gebunden sind, oder
Glucosaminmolektile die mit der besagten Verzweigungseinheit verbunden sind, oder
monomere / oligomere Zuckerreste mit spezifischen Adhäsionspunkten für Selektine auf spezifischen Zellen, so dass der Komplex auf besagte spezifische Zellen targetiert ist, oder
ein Cyclodextrin.

3. Ein Komplex nach einem der Ansprüche 1 oder 2, wobei die besagte therapeutische Verbindung aus einem Photosensibilator (Photosensitizer) oder einem chemotherapeutischen Wirkstoff besteht, wobei sich der besagte Photosensitizer während der Dauer der Aktivierung vorzugsweise nahe an der besagten Membran befinden sollte, um die Wirksamkeit des besagten Photosensitizers zu verstärken im Vergleich zu einem ähnlichen Photosensitizer ohne die besagte Fallschirmstruktur.

4. Ein Komplex nach einem der Ansprüche 1 bis 3, wobei die besagte Fallschirmstruktur entweder direkt oder mittels eines Abstandshalters mit der besagten therapeutischen Verbindung verbunden ist, wobei besagter Abstandshalter vorzugsweise aus β-Aminosäuren, γ-Aminobuttersäure oder Polyaminosäuren besteht, und wobei Typ und Anzahl des besagten eingesetzten Abstandshalters den Abstand des besagten therapeutischen Wirkstoffs zu den Zellmembranen oder seine Lokalisierung im Zellinnern bestimmen.

5. Ein Komplex nach Anspruch 4, wobei der besagte Abstandshalter vorzugsweise aus einem aliphatischen, aromatischen oder heterozyklischen Molekül oder einer Aminosäurensequenz besteht.

6. Ein Komplex nach Anspruch 5, wobei die besagte Aminosequenz eine durch Enzyme spaltbare Bruchstelle besitzt.

7. Ein Komplex nach einem der Ansprüche 4 bis 6, wobei durch den Einsatz besagter, nach Anzahl oder Typ unterschiedlicher Abstandshalter zur Anbindung der besagten therapeutischen Verbindung an die besagte Fallschirmstruktur den besagten Komplex in subzelluläre Bereiche in einer bestimmten Distanz von der Oberfläche der besagten Bereiche befördert werden.

8. Ein Komplex nach einem der Ansprüche 1 bis 7, wobei die besagten Fallschirmstrukturen mit Signalen zum Targeting des besagten Komplexes in ein bestimmtes Gewebe oder einen bestimmten Zelltyp eines Organismus modifiziert sind.

9. Ein Komplex nach Anspruch 8, wobei die besagten modifizierten Signale Brückenstrukturen wie z.B. ein Biotin-Avidin System enthalten.

10. Ein Komplex nach einem der Ansprüche 1 bis 9, wobei besagter Komplex zur Zerstörung von Zellen genutzt werden kann, und wobei besagte Zellen prokaryotisch-vorzugsweise Bakterien - oder eukaryotisch - vorzugsweise menschliche oder ticrische Zellen - sind.

11. Die Nutzung eines Komplexes mit mindestens einer Fallschirmstruktur und mindestens einem Photosensitizer bei der Herstellung eines Medikamentes zur Behandlung und selektiven Zerstörung von eukaryotischen oder prokaryotischen Zellen in folgenden Schritten:
a. Verabreichen des besagten Komplexes, wobei der besagte Komplex eine Fallschirmstruktur und einen Photosensitizer enthält; und
b. abwarten, bis besagter Komplex sich selektiv an den Zellmembranen oder an der bestimmten Position im Zellinnern lokalisiert; und
c. Bestrahlen eines Bereiches, in den der besagte Komplex appliziert wurde, für eine bestimmte Zeitdauer und mit einer bestimmten Intensität zwecks Aktivierung des besagten Photosensitizers, wobei besagter Zeitraum und Intensität für die selektive Zerstörung der gewünschten Zellen ausreichend sind.

## Revendications

1. Un complexe pour le transport et l'application de médicaments aux membranes cellulaires, ou à l'intérieur de la cellule à une certaine distance des membranes, qui consiste en:
Une structure à parachute **(106)** d'un diamètre d'action **(108)** défini, qui empêche ladite structure de pénétrer la membrane d'une cellule **(100);**
Où ladite structure à parachute comprend des résidus hydrophiles qui sont des résidus de sucre ayant ledit diamètre d'action **(108);** et
Un composé thérapeutique **(102)** apte à pénétrer la membrane de ladite cellule **(100)** et, en même temps, à continuer à faire partie dudit complexe.

2. Un complexe selon la revendication 1, dont ledit diamètre d'action peut être acquis par :
une unité de branchement à laquelle lesdits résidus sont accouplés, ou
des molécules de glucosamine accouplés à ladite unité de branchement, ou
des résidus sucrés monomères / oligomères avec des points spécifiques d'adhésion pour des sélectines sur des cellules spécifiques, afin que le complexe soit ciblé sur lesdites cellules spécifiques, ou
une cyclodextrine.

3. Un complexe selon l'une des revendications 1 ou 2, où ledit composé thérapeutique se compose d'un photosensibilisant ou d'une substance chimiothérapeutique, ledit photosensibilisant devant être, pendant la période d'activation, de préférence le plus proche possible de ladite membrane afin de renforcer l'efficacité dudit photosensibilisant par rapport à un photosensibilisant similaire sans ladite structure à parachute.

4. Un complexe selon l'une des revendications 1 à 3, où ladite structure à parachute est accouplée, directement ou à l'aide d'un segment espaccur, audit composé thérapeutique, et où ledit segment espaceur consiste de préférence en des bêta-acides aminés, des acides gamma-amino-butyriques ou des polyaminoacides, et où le type et le nombre dudit segment espaceur utilisé définissent la distance de ladite substance aux membranes cellulaires ou sa localisation à l'intérieur de la cellule.

5. Un complexe selon la revendication 4, où ledit segment espaceur se compose de préférence d'une molécule aliphatique, aromatique où hétérocyclique ou d'une séquence d'acides aminés.

6. Un complexe selon la revendication 5, où ladite séquence d'acides aminés possède un point de rupture fissile par des enzymes.

7. Un complexe selon l'une des revendications 4 à 6, où, par l'utilisation de différents types ou nombres desdits segments espaceurs pour l'accouplement dudit composé thérapeutique à ladite structure à parachute, ledit complexe est transporté dans des zones subcellulaires à une certaine distance de la surface desdites zones.

8. Un complexe selon l'une des revendications 1 à 7, où lesdites structures à parachute sont modifiées avec des signaux pour le ciblage dudit complexe dans un certain tissu où dans un certain type cellulaire d'un organisme.

9. Un complexe selon la revendication 8, où lesdits signaux modifiés contiennent des structures de pont comme p.ex. un système biotine-avidine.

10. Un complexe selon l'une des revendications 1 à 9, où ledit complexe peut être utilisé pour la destruction de cellules et où lesdites cellules sont procaryotiques - de préférence des bactéries - ou eucaryotiques - de préférence des cellules humaines ou animales.

11. L'utilisation d'un complexe avec au moins une structure à parachute et au moins un photosensibilisant lors de la fabrication d'un médicament pour le traitement et la destruction sélective de cellules eucaryotiques ou procaryotiques, comprenant les étapes suivantes:
a. administrer ledit complexe, le complexe en question comprenant une structure à parachute et un photosensibilisant; et
b. attendre jusqu'à ce que ledit complexe se localise sélectivement au niveau des membranes cellulaires ou à la position déterminée à l'intérieur de la cellule; et
c. irradiation d'une zone dans laquelle ledit complexe avait été appliqué pour une période et une intensité déterminées afin d'activer ledit photosensibilisant, la période et l'intensité étant suffisantes pour la destruction sélective des cellules choisies.
